(19) Europäisches Patentamt / European Patent Office / Office européen des brevets

(11) **EP 4 160 523 B1**

(12) **EUROPÄISCHE PATENTSCHRIFT**

(45) Veröffentlichungstag und Bekanntmachung des Hinweises auf die Patenterteilung:
**29.10.2025  Patentblatt 2025/44**

(21) Anmeldenummer: **21199847.1**

(22) Anmeldetag: **29.09.2021**

(51) Internationale Patentklassifikation (IPC):
**G06T 7/00** (2017.01)

(52) Gemeinsame Patentklassifikation (CPC):
**G06T 7/0012;** G06T 2207/10116;
G06T 2207/20084; G06T 2207/30068;
G06T 2207/30096

(54) **COMPUTER-IMPLEMENTIERTES VERFAHREN ZUM ERMITTELN EINER AUFFÄLLIGEN STRUKTUR**

COMPUTER-IMPLEMENTED METHOD FOR IDENTIFYING A CONSPICUOUS STRUCTURE

PROCÉDÉ MIS EN OEUVRE PAR ORDINATEUR PERMETTANT DE DÉTERMINER UNE STRUCTURE VISIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.04.2023  Patentblatt 2023/14**

(73) Patentinhaber: **Siemens Healthineers AG**
**91301 Forchheim (DE)**

(72) Erfinder:
• **Hörnig, Mathias**
**91096 Möhrendorf (DE)**
• **Hümmer, Christian**
**96215 Lichtenfels (DE)**

(74) Vertreter: **Siemens Healthineers Patent Attorneys**
**Postfach 22 16 34**
**80506 München (DE)**

(56) Entgegenhaltungen:
**US-A1- 2019 021 677     US-A1- 2021 287 799**
**US-B2- 10 957 079**

## Beschreibung

Die Erfindung betrifft ein computer-implementiertes Verfahren zum Ermitteln einer auffälligen Struktur und ein Röntgensystem, ein Computerprogrammprodukt und ein computerlesbares Medium.

Computerunterstützte Diagnose (engl.: Computer-Aided Diagnosis, Abk.: CAD), welche künstliche Intelligenz (Abk.: KI; engl.: Artificial Intelligence, Abk.: AI) verwendet, ist ein bekanntes Werkzeug, um verdächtige Bereiche und/oder Wahrscheinlichkeiten des Vorliegens von dedizierten Funden in medizinischen Bildern zu identifizieren bzw. abzuschätzen. Beispiele sind bekannt aus Santos, M. K., Ferreira Júnior, J. R., Wada, D. T. , Tenório, A., Barbosa, M., & Marques, P. (2019). Artificial intelligence, machine learning, computer-aided diagnosis, and radiomics: advances in imaging towards to precision medicine. Radiologia brasileira, 52(6), 387-396. https://doi.org/10.1590/0100-3984.2019.0049 und L. Oakden-Rayner: "The Rebirth of CAD: How Is Modern AI Different from the CAD We Know?, Radiology: Artificial Intelligence 2019; 1(3):e180089. https://doi.org/10.1148/ryai.2019180089.

Die Verwendung von KI-basierten CAD-Algorithmen ist bisher auf medizinische Bilder limitiert, die mit einschichtigen Röntgen-(Flach-)Detektoren aufgenommen wurden, z.B. mittels a-Si-Technologie.

Aus der Druckschrift US 7,671,342 B2 ist ein Röntgendetektor bestehend aus mehreren Schichten, z. B. aus mehreren Schichten von Leuchtstoffschirmen und/oder Detektoren bzw. Detektionsschichten, bekannt. Einige Röntgenstrahlen, die eine Schicht durchdringen, werden in einer anderen Schicht erfasst oder in Lichtenergie umgewandelt. So befindet sich beispielsweise ein Phosphorschirm vor und ein weiterer hinter der Detektorschaltung. Das in jedem der Leuchtstoffschirme erzeugte Licht wird von derselben Detektorschaltung erfasst. Ein weiteres Beispiel sind mehrere Schichten von Leuchtstoffschirmen und zugehörige Detektorschaltungen. Einige Röntgenstrahlen, die eine Schicht durchdringen, können in einer anderen Schicht erfasst werden. Es können sowohl hochenergetische Röntgenstrahlen, die mit Megavoltage-Quellen verbunden sind, als auch Röntgenstrahlen mit niedrigerer oder höherer Energie erfasst werden. Ferner ist aus dem Artikel "KA IMAGING'S X-RAY DETECTOR ALLOWS ANY X-RAY SYSTEM TO BE UPGRADED TO DUAL-ENERGY" (https://www.kaimaging.com/blog/ka-imagings-x-raydetector-allows-any-x-ray-system-to-be-upgraded-to-dual-energy/) ein Dual-Energy-Röntgendetektor bekannt.

Die Röntgenstrahlung oder die Photonen können in direktkonvertierenden Röntgendetektoren durch ein geeignetes Konvertermaterial in elektrische Pulse umgewandelt werden. Als Konvertermaterial können beispielsweise CdTe, CZT, CdZnTeSe, CdTeSe, CdMnTe, InP, TlBr2, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung durch Zählen der elektrischen Pulse, welche durch die Absorption von Röntgenphotonen im Konvertermaterial ausgelöst werden, gemessen. Die Höhe des elektrischen Pulses ist in der Regel proportional zur Energie des absorbierten Röntgenphotons. Dadurch kann eine spektrale Information durch den Vergleich der Höhe des elektrischen Pulses mit einem Schwellwert extrahiert werden.

Aus klinischer Sicht ermöglichen es Mehrschichtdetektoren aus aufgezeichneten Röntgeninformationen unterschiedlicher Energie zu identischen Zeitpunkten eine Reihe von diagnostischen Bildern zu erstellen. Die Ausnutzung mehrerer Detektorschichten ermöglicht eine quantitative Analyse von Materialien mittels energieauflösender ("spektraler") Messungen und ermöglicht eine genaue Detektion potenzieller (Be-)Funde durch die Bereitstellung diagnostischer Bilder für Materialien mit unterschiedlichen Eigenschaften im Bild (z. B. Unterscheidung von Knochen und Weichgewebe). Der erzeugte Bildsatz (z. B. zwei Bilder für ein Zwei-Energie-System) wird vom klinischen Experten inspiziert, um ein Urteil über den Gesundheitszustand des Patienten und mögliche Behandlungen zu fällen.

Aus algorithmischer Sicht kann komplementäre Information in Eingangsdaten für KI-basierte CAD-Algorithmen verwendet werden, jedoch basieren bisher die komplementären Daten auf einem Eingangsbild ohne eine Möglichkeit mehrere physikalische Detektionsschichten zu berücksichtigen.

KI-basierte Algorithmen können verwendet werden, um Sinogramme von Dual-Energy-CT mit monoenergetischen Sinogrammen zu verbinden (vgl. Yang, H., Cong, W., & Wang, G. (2017). Deep learning for dual-energy X-ray computed tomography. In Proceedings of The 14th International Meeting on Fully Three-Dimensional Image Reconstruction in Radiology and Nuclear Medicine (pp. 864-869)9, um eine Rauschreduktion durchzuführen (vgl. Lee, S., Choi, Y.H., Cho, Y.J. et al. Noise reduction approach in pediatric abdominal CT combining deep learning and dual-energy technique. Eur Radiol 31, 2218-2226 (2021). https://doi.org/10.1007/s00330-020-07349-9), Dual-Energy-Daten aus Single-Energy-Daten abzuschätzen (vgl. Tianling Lyu, Wei Zhao, Yinsu Zhu, Zhan Wu, Yikun Zhang, Yang Chen, Limin Luo, Shuo Li, Lei Xing, Estimating dual-energy CT imaging from single-energy CT data with material decomposition convolutional neural network, Medical Image Analysis, Volume 70, 2021, 102001, ISSN 1361-8415, https://doi.org/10.1016/j.media.2021.102001.), und um nichtkontrastverstärkte Bilder abzuschätzen (vgl. Steffen Bruns et al: "Deep Learning from Dual-Energy Information for Whole-Heart Segmentation in Dual-Energy and Single-Energy Non-Contrast-Enhanced Cardiac CT", https://arxiv.org/abs/2008.03985) .

Die US 10,957,079 B2 offenbart ein Verfahren zum Bestimmen einer bösartigen Veränderung in einem Mammographie-Bild, wobei basierend auf einem Einzelkanal Mammographie-Bild ein Mehrkanal Mammographie-Bild bestimmt wird und die bösartige Veränderung durch Anwenden eines Klassifizierers auf das Mehrkanal Mammographie-Bild bestimmt wird.

Die US 2019/0021677 A1 beschreibt ein Verfahren zum Bestimmen einer Verletzung. Dabei wird ein Dual-Energy Bild von einem CT Scanner vorverarbeitet, wobei ein virtuelles, kalziumfreies Bild bestimmt wird. Auf dieses virtuelle, kalziumfreie Bild wird ein Deep Learning Algorithmus angewendet, um die Verletzung zu bestimmen.

Der Erfindung liegt das Problem zugrunde, die Genauigkeit eines KI-basierten CAD-Algorithmus basierend auf den Aufnahmedaten zu verbessern, um eine höhere Zuverlässigkeit bei den CAD-Funden zu ermöglichen.

Es ist Aufgabe der Erfindung, eine Vorrichtung X und ein Verfahren X anzugeben, welche eine Verbesserung der Genauigkeit eines KI-basierten CAD-Algorithmus ermöglichen.

Die Aufgabe wird erfindungsgemäß gelöst durch ein computer-implementiertes Verfahren zum Ermitteln einer auffälligen Struktur nach Anspruch 1, eine Ermittlungseinheit nach Anspruch 11, ein Röntgensystem nach Anspruch 12, ein Computerprogrammprodukt nach Anspruch 13 und ein computerlesbares Medium nach Anspruch 14.

Die Erfindung betrifft ein computer-implementiertes Verfahren zum Ermitteln einer auffälligen Struktur in einem Untersuchungsbereich in Verbindung mit einer Röntgenaufnahme eines Röntgensystems aufweisend folgende Schritte des Empfangens, des Anwendens und des Bereitstellens. Im Schritt des Empfangens werden Eingangsdaten empfangen, wobei sich die Eingangsdaten auf einen Röntgenaufnahmedatensatz der Röntgenaufnahme aufweisend mehrere Datenkanäle beziehen. Im Schritt des Anwendens wird eine trainierte Funktion auf die Eingangsdaten angewendet, wobei die trainierte Funktion auf einem maschinellen Lernverfahren basiert, wobei die trainierte Funktion auf mindestens zwei Datenkanäle hinsichtlich eines Ermittelns der auffälligen Struktur angewendet wird, und wobei Ausgangsdaten erzeugt werden. Im Schritt des Bereitstellens werden Ausgangsdaten bereitgestellt, wobei die Ausgangsdaten eine auffällige Struktur des Untersuchungsbereichs umfassen.

Das Ermitteln kann auch als Finden, Bestimmen, Bewerten oder Bereitstellen bezeichnet werden. Die auffällige Struktur kann auch als diagnostisch relevante Eigenschaft bezeichnet werden. Die auffällige Struktur kann beispielsweise ein verdächtiger Bereich oder die Wahrscheinlichkeit für die Existenz eines dedizierten Funds bzw. Befunds sein.

Die Erfinder haben erkannt, dass mittels energieauflösender Röntgendetektoren die Genauigkeit von KI-basierten CAD-Algorithmen mittels der komplementären Informationen, aufgenommen in beispielsweise verschiedenen Detektionsschichten oder Detektionskanälen, verbessert werden können.

Das Röntgensystem, beispielsweise mit Anwendung in der Computertomographie, der Angiographie, Mammographie, Fluoroskopie oder der Radiographie, kann einen zählenden direktkonvertierenden Röntgendetektor oder einen Mehrschichtröntgendetektor umfassen. Sowohl der zählende Röntgendetektor als auch der Mehrschichtröntgendetektor kann mehrere Datenkanäle als Ausgang umfassen. Die trainierte Funktion kann an einen Röntgendetektor, insbesondere dessen Eigenschaften, angepasst sein.

Die Erfinder schlagen vor, die in den verschiedenen Detektionsschichten oder Datenkanälen aufgenommen Informationen simultan für KI-basierte CAD-Algorithmen bzw. die trainierte Funktion auszunutzen.

Das Verfahren kann dabei folgende Schritte umfassen. Mittels des Mehrschichtröntgendetektors bzw. des zählenden Röntgendetektors können N, insbesondere zweidimensionale, Aufnahmebilddatensätze bzw. ein Röntgenaufnahmebilddatensatz mit N Kanälen in einem Schritt der Röntgenaufnahme erzeugt werden. Die Aufnahmebilddatensätze bzw. der Röntgenaufnahmebilddatensatz können entweder vom Röntgendetektor selbst erzeugt werden oder es können zusätzliche Aufnahmebilddatensätze basierend auf einer Rekombination von Ausgangsinformationen, insbesondere in verschiedenen Datenkanälen, berechnet bzw. erzeugt werden. Die N Aufnahmebilddatensätze bzw. der Röntgenaufnahmebilddatensatz mit N Kanälen können als Eingangsdaten für die trainierte Funktion verwendet werden. Im Schritt des Empfangens werden Eingangsdaten empfangen, wobei sich die Eingangsdaten auf einen Röntgenaufnahmedatensatz der Röntgenaufnahme aufweisend mehrere Datenkanäle bzw. mehrere Aufnahmebilddatensätze beziehen. Beispielsweise können mit einem Mehrschichtröntgendetektor bzw. einem zählenden Röntgendetektor N Detektorbilder bzw. ein Röntgenaufnahmedatensatz mit N Kanälen aufgenommen werden. Diese N Detektorbilder bzw. der Röntgenaufnahmedatensatz mit den N Kanälen können als Eingangsdaten für die trainierte Funktion, insbesondere einen KI-basierten CAD-Algorithmus verwendet werden.

Im Schritt des Anwendens wird eine trainierte Funktion auf die Eingangsdaten angewendet. Die trainierte Funktion basiert auf einem maschinellen Lernverfahren. Im Allgemeinen kann eine trainierte Funktion kognitive Funktionen, welche man mit einem menschlichen Gehirn in Verbindung bringen kann, nachempfinden. Insbesondere beim Trainieren basierend auf Trainingsdaten kann die trainierte Funktion an neue Randbedingungen angepasst werden und Muster erkennen und extrapolieren.

Im Allgemeinen können Parameter einer trainierten bzw. trainierbaren Funktion mittels Trainings angepasst werden. Es kann insbesondere ein überwachtes, teilüberwachtes, unüberwachtes, verstärkendes Lernen oder aktives Lernen verwendet werden. Ferner kann sogenanntes "Representation Learning" verwendet werden. Insbesondere können die Parameter der trainierten Funktion iterativ in mehreren Schritten des Trainings angepasst werden.

Insbesondere kann eine trainierte Funktion ein neuronales Netzwerk, eine Support-Vektor-Maschine, einen Entscheidungsbaum oder ein Bayes'sches Netz umfassen, und/oder die trainierte Funktion kann auf sogenanntem k-means clustering, Q-learning, genetischen Algorithmen und/oder Assoziationsregeln basieren. Insbesondere kann das neuronale Netzwerk ein sogenanntes Deep Neural Network, ein Convolutional Neural Network oder ein Convolutional Deep Neural Network sein. Ferner kann das neuronale Netzwerk ein sogenanntes adversarial network, ein deep adversarial network und/oder ein generative adversarial network sein.

Die trainierte Funktion kann auf einem überwachten Lernverfahren basieren. Die trainierte Funktion kann auf einem maschinellen Lernverfahren basieren. Die trainierte Funktion kann insbesondere auf einem neuronalen Netz basieren. Es können vor allem annotierte Eingangs- und Ausgangstrainingsdaten zum Trainieren verwendet werden. Es kann bevorzugt ein Deep Learning Verfahren verwendet werden. Alternativ kann ein maschinelles Lernverfahren verwendet werden. Die Features können manuell oder bevorzugt maschinell extrahiert werden. Als neuronales Netz kann Feed forward neural networks, Recurrent Neural Networks (RNN) oder bevorzugt Convolutional Neural Networks (CNN) verwendet werden. Vorteilhaft können mittels des überwachten Lernverfahrens Zusammenhänge zwischen den Eingangs- und Ausgangstrainingsdaten erschlossen werden und auf Eingangsdaten angewendet werden.

Maschinelles Lernen im Sinne der Erfindung umfasst eine computer-implementierte Technik, bei der ein Algorithmus auf Basis von bestehenden Daten Muster bzw. Gesetzmäßigkeiten erkennt und unter Anwendung derselben in Bezug auf unbekannte, neue Daten eigenständig Lösungen ableitet. Voraussetzung für eine eigenständige Lösungsfindung ist eine Trainingsphase, in der ein Algorithmus des Maschinen-Lernens auf einen bekannten, definierten und zumeist sehr großen Datenbestand angewandt wird, um diejenigen Regeln bzw. Vorhersagen zu finden, die eine gewünschte Ausgabe bzw. ein gewünschtes Ergebnis erzielen. Das Training kann als überwachtes oder unüberwachtes Training ausgebildet sein, wobei in der ersten Variante dem Algorithmus Werte-Paare in Form von Eingangstrainingsdaten und dazu gehörigen, korrekten Ausgangstrainingsdaten präsentiert werden, wohingegen in der zweiten Variante der Algorithmus sich basierend auf den Eingangstrainingsdaten eigenständig derart selbst anpassen muss, dass er die korrekten Ausgangstrainingsdaten liefert.

Besonders vorteilhaft ist der Algorithmus des Maschinen-Lernens als künstliches neuronales Netz ausgebildet. Ein künstliches neuronales Netz orientiert sich am Aufbau eines biologischen, neuronalen Netzes wie bspw. einem menschlichen Gehirn. Ein künstliches neuronales Netz umfasst zwischen einer Eingangs- und einer Ausgangsschicht bevorzugt eine Vielzahl von weiteren Schichten jeweils umfassend wenigstens einen Knoten. Jeder Knoten entspricht dabei einer Verarbeitungseinheit, analog einem biologischen Neuron. Knoten innerhalb einer Schicht des Netzes können über gerichtete Verbindungen oder Kanten (edges) mit Knoten anderer Schichten verbunden sein. Die Verbindungen definieren den Datenfluss innerhalb des Netzes. Jeder Knoten repräsentiert folglich eine Operation, die auf die Eingangsdaten angewendet wird. Ferner verfügt jeder Knoten bzw. jede seiner Verbindungen über mindestens einen Gewichtungsparameter (weight). Über diesen Gewichtungsparameter bzw. diese Gewichtungsparameter wird der Einfluss bzw. die Wichtigkeit der Ausgabe eines Knotens als Eingabewert für einen Empfängerknoten definiert. In der Trainingsphase, die bevorzugt als überwachtes Lernen ausgeführt wird, 'lernt' das Künstliche Neuronale Netz anhand der Trainingsdaten die Gewichtungsparameter für alle Knoten bzw. Verbindungen und passt diese so lange an, bis die Ausgabeschicht des Netzes die korrekten Ausgangsdaten bzw. Ausgangstrainingsdaten liefert.

Die erfindungsgemäße Vorgehensweise beruht ferner auf der Erkenntnis, dass ein trainierter Algorithmus des Maschinen-Lernens im Rahmen seines Trainings einen festen Zusammenhang zwischen Eingangsdatensätzen, hier Eingangstrainingsdaten bezogen auf einen Röntgenaufnahmedatensatz mit mehreren Datenkanälen, und Ausgabewerten, hier eine auffällige Struktur des Untersuchungsbereichs, herstellt.

In einer Ausführungsform kann die trainierte Funktion auf einem sogenannten Deep Learning-Verfahren basieren. Beispielsweise kann ein 'convolutional neural network', auch faltendes neuronales Netz genannt, verwendet werden. Mit anderen Worten wird gemäß dieser Ausführung mittels eines Algorithmus des Maschinen-Lernens zunächst eine Merkmalsextraktion durchgeführt, und anschließend eine sogenannte Klassifikation oder Regression, wobei die identifizierten Merkmale bezogen auf die Datenkanäle einer auffälligen Struktur des Untersuchungsbereichs zugeordnet werden können. Bei einer Klassifikation können die Wahrscheinlichkeiten zwischen 0 und 1 liegen. Bei einer Regression können kontinuierliche Werte verwendet werden, beispielsweise Koordinaten im Bild zur Beschreibung einer suspekten Region oder einer auffälligen Struktur. Alternativ zu einem faltenden neuronalen Netz können auch Lange Kurzzeit-Gedächtnis (LSTM long short-term memory) Netze oder rekurrente neuronale Netze (RNN) zum Einsatz kommen, welche im Gegensatz zu den vorher genannten rückwärts gerichtete Feedback-Schleifen innerhalb der versteckten Netzschichten aufweisen.

Die trainierte Funktion kann auf mindestens zwei Datenkanäle bzw. zwei Aufnahmebilddatensätze hinsichtlich eines Ermittelns der auffälligen Struktur angewendet werden. Es werden Ausgangsdaten erzeugt. Im Schritt des Bereitstellens werden die Ausgangsdaten bereitgestellt, wobei die Ausgangsdaten eine auffällige Struktur des Untersuchungsbereichs umfassen.

Vorteilhaft können simultan bzw. parallel oder gleichzeitig die komplementären Informationen, aufgenommen in den verschiedenen Schichten des Mehrschichtröntgendetektors oder den verschiedenen Energiekanälen des zählenden Röntgendetektors als Eingangsdaten für die trainierte Funktion verwendet werden. Vorteilhaft können simultan bzw. parallel oder gleichzeitig spektrale oder materialauflösende Informationen oder Informationen abgeleitet bzw. basierend auf einer Rekombination von Detektorausgangsbildern bzw. Aufnahmedatensätzen als Eingangsdaten für die trainierte Funktion verwendet werden.

Gemäß einem Aspekt der Erfindung wird für jeweils einen Datenkanal ein, insbesondere zweidimensionaler, Aufnahmebilddatensatz bereitgestellt. Für eine Schicht des Mehrschichtröntgendetektors kann ein zweidimensionaler Aufnahmebilddatensatz bereitgestellt werden. Der Mehrschichtröntgendetektor kann eine Matrix von Detektionselement in jeder Schicht aufweisen. Die Detektionselemente können in den verschiedenen Schichten gleich oder verschieden groß ausgebildet sein. Im Fall von verschieden groß ausgebildeten Detektionselementen in den verschiedenen Schichten kann ein Rebinning angewendet werden, bevor die trainierte Funktion auf die Eingangsdaten angewendet wird. Eine Schicht kann, insbesondere genau, einen Datenkanal bezeichnen.

Für einen Energiekanal eines zählenden Röntgendetektors kann ein zweidimensionaler Aufnahmebilddatensatz bereitgestellt werden. Der zählende Röntgendetektor kann eine Matrix von Detektionselement für jeden Energiekanal aufweisen. Die Detektionselemente können in den verschiedenen Energiekanälen gleich oder verschieden groß ausgebildet sein. Im Fall von verschieden groß ausgebildeten Detektionselementen in den verschiedenen Energiekanälen kann ein Rebinning angewendet werden, bevor die trainierte Funktion auf die Eingangsdaten angewendet wird. Ein Energiekanal kann, insbesondere genau, einen Datenkanal bezeichnen. Vorteilhaft können die spektralunterschiedlichen bzw. -komplementären Informationen für eine verbesserte Erkennung von einer auffälligen Struktur im Untersuchungsbereich genutzt werden.

Gemäß einem Aspekt der Erfindung beziehen sich die Datenkanäle auf voneinander verschiedene Datensätze aus der Gruppe aus: einem Weichteildatensatz, einem Knochendatensatz, einem nativen Bilddatensatz, einem Kontrastmitteldatensatz, einem Energiebereichsdatensatz oder mehreren voneinander verschiedenen Energiebereichsdatensätzen.

Der Weichteildatensatz oder der Knochendatensatz kann basierend auf einer Materialzerlegung und damit einer Rekombination von Informationen aus verschiedenen Datenkanälen erzeugt werden. Der Kontrastmitteldatensatz kann basierend auf einer Aufnahme mit Kontrastmittel, ggf. unter Anwendung von zwei verschiedenen Röntgenspektren bei der Aufnahme, erzeugt werden. Der native Bilddatensatz kann beispielsweise die Information aus mehreren oder allen Datenkanälen umfassen und insbesondere ein "normales" Röntgenbild darstellen.

Der Energiebereichsdatensatz kann sich auf einen Energiekanal eines zählenden Röntgendetektors beziehen. Der Energiebereichsdatensatz kann sich auf eine Schicht eines Mehrschichtröntgendetektors beziehen. Mehrere voneinander verschiedene Energiebereichsdatensätze können insbesondere disjunkte Energiebereich betreffen. Mehrere voneinander verschiedene Energiebereichsdatensätze können überlappende Energiebereiche betreffen. Vorteilhaft kann eine spektrale Information für die Bestimmung von auffälligen Strukturen genutzt werden.

Gemäß einem Aspekt der Erfindung ist der Röntgenaufnahmedatensatz mit einem Mehrschichtröntgendetektor oder einem photonenzählender Röntgendetektor aufgenommen. Der photonenzählende Röntgendetektor kann auch als zählender Röntgendetektor bezeichnet werden. Vorteilhaft können spektrale Informationen mittels einer Röntgenaufnahme aufgenommen werden.

Gemäß einem Aspekt der Erfindung umfassen die Eingangsdaten:

- komplementäre Informationen in den Datenkanälen,
- spektrale oder materialauflösende Informationen, oder/und
- Informationen basierend auf einer Rekombination von Bilddaten der Datenkanäle.

Insbesondere bei Verwendung von einem Weichteildatensatz, einem Knochendatensatz, einem nativen Bilddatensatz (insbesondere abzüglich einer Kontrastmittelinformation), einem Kontrastmitteldatensatz, einem Energiebereichsdatensatz oder mehreren voneinander verschiedenen Energiebereichsdatensätzen kann eine komplementäre Information eine spektrale oder materialauflösende Information und/oder eine Information basierend auf einer Rekombination von Bilddaten der Datenkanäle von der trainierten Funktion genutzt werden, um eine verbesserte Bestimmung von einer auffälligen Struktur zu ermöglichen. Die materialauflösende Information kann sich beispielsweise auf Weichteil- und Knochendatensatz beziehen. Die komplementäre Die spektrale Information kann sich beispielsweise auf einen Energiebereichsdatensatz oder auf mehrere voneinander verschiedene Energiebereichsdatensätze beziehen. Die Rekombination von Bilddaten der Datenkanäle kann sich beispielsweise auf eine nativen Bilddatensatz beziehen.

Gemäß einem Aspekt der Erfindung wird die trainierte Funktion gleichzeitig auf die mindestens zwei Datenkanäle angewendet. Es kann insbesondere eine trainierte Funktion, d.h. beispielsweise ein neuronales Netz, auf zwei Datenkanäle angewendet werden. Alternativ kann ein eine erste trainierte Funktion auf einen ersten Datenkanal und eine zweite trainierte Funktion auf einen zweiten Datenkanal, insbesondere gleichzeitig bzw. parallel, angewendet werden. Vorteilhaft können trotz der erhöhten Datenmenge in Bezug auf die Eingangsdaten schnell Ausgangsdaten erzeugt werden.

Gemäß einem Aspekt der Erfindung basiert die trainierte Funktion auf einem Deep-Learning-Verfahren für RGB-Bilder. Ein Beispiel für die Anwendung des erfindungsgemäßen Verfahrens ist die Verwendung eines Deep-Learning-Modells (DL-Modell) bzw. eines Deep-Learning-Verfahren, insbesondere eines, welches für RGB-Bilder bzw. RGB-Bilddaten optimiert ist. Das Deep-Learning-Verfahren kann bezüglich RGB-Bilddaten optimiert sein, um Information aus den drei Eingangskanälen zu extrahieren. Anstatt das Modell anzupassen, um nur ein Bild zu verarbeiten oder das gleiche Bild in die drei RGB-Kanäle zu kopieren, vgl. dazu:

- M. Chen, "Automated bone age classification with deep neural networks", 2016, http://cs231n.stan ford.edu/reports/2016/pdfs/310_Report.pd f
- H. Lee, "Fully automated deep learning systems for bone age assessment", J Digit Imaging 2017, https:// link.springer.com/article/10.1007/ s10278-017-9955-8
- S. Guendel et al.: "Learning to Recognize Abnormalities in Chest X-Rays with Location-Aware Dense Networks", CIARP 2018, https://arxiv.org/abs/ 1803.04565
- Z. Li et al.: "Thoracic Disease Identification and Localization with Limited Supervision", CVPR 2018, https://arxiv.org/abs/1711.06373,

haben die Erfinder festgestellt, dass eine es eine vielversprechende Alternative ist, die Röntgenaufnahmedaten aufweisend mehrere Datenkanäle, insbesondere eines Mehrschichtröntgendetektors oder eines photonenzählenden Röntgendetektors, dabei als Eingangsdaten des DL-Modells bzw. der trainierten Funktion auszunutzen. Ein existierendes oder bekanntes RGB-Modell kann die Informationen aus drei Aufnahmebilddatensätzen bzw. drei Datenkanälen, insbesondere eines Mehrschichtröntgendetektors oder eines photonenzählenden Röntgendetektors, extrahieren. Beispielsweise kann basierend auf dem Röntgenaufnahmedatensatz ein erster Aufnahmebilddatensatz bzw. ein erster Datenkanal bezüglich Weichteilen bzw. Weichgewebe, ein zweiter Aufnahmebilddatensatz bzw. zweiter Datenkanal bezüglich Knochenstrukturen und ein dritter Aufnahmebilddatensatz bzw. Datenkanal bezüglich eines nativen Bilddatensatzes erzeugt und als Eingangsdaten für das RGB-Modell bereitgestellt werden. Der erste Datenkanal kann beispielsweise für den R-Kanal des RGB-Modells genutzt werden, der zweite Datenkanal für den G-Kanal und der dritte Datenkanal für den B-Kanal. Vorteilhaft kann ein existierendes Deep-Learning-Verfahren verwendet werden.

Gemäß einem Aspekt der Erfindung werden drei Datenkanäle verwendet. Insbesondere im Hinblick auf ein RGB-(DL-)Modell können der R-, G- und B-Kanal genutzt werden.

Gemäß einem Aspekt der Erfindung wird vor Anwenden der trainierten Funktion eine Bildverarbeitung auf den Röntgenaufnahmedatensatz angewendet. Die Bildverarbeitung kann Schritte zur Rekombination von Informationen der Datenkanäle umfassen. Die Bildverarbeitung kann Rauschkorrektur, Intensitätsanpassung oder Artefaktkorrektur umfassen. Vorteilhaft kann durch eine Bildverarbeitung, insbesondere eine Bildvorverarbeitung, die gleiche trainierte Funktion, für beispielsweise verschiedene Röntgendetektoren bzw. deren Röntgenaufnahmedatensätze verwendet werden. Durch die Bildverarbeitung kann der Röntgenaufnahmedatensatz eines ersten Röntgendetektors derart angepasst werden, dass er die gleichen Bildeigenschaften wie ein Röntgenaufnahmedatensatz eines zweiten Röntgendetektors aufweisen kann. Vorteilhaft kann eine Anpassung der trainierten Funktion auf einen anderen Röntgendetektor vermieden werden.

Gemäß einem Aspekt der Erfindung umfassen die Ausgangsdaten als auffällige Struktur eine Position einer Läsion, eine Position eines Mikrokalks, eine Position einer Landmarke, einen Abstand oder einen Winkel. Die Ausgangsdaten können eine Koordinate innerhalb des Röntgenaufnahmedatensatzes umfassen. Die Ausgangsdaten können eine Fläche, einen Abstand oder einen Winkel, insbesondere mittels Koordinaten innerhalb des Röntgenaufnahmedatensatzes, umfassen. Die Ausgangsdaten können insbesondere eine Lage bzw. Position und Ausdehnung einer auffälligen Struktur umfassen. Vorteilhaft kann mittels der Ausgangsdaten die auffällige Struktur im Röntgenaufnahmedatensatzes dargestellt werden. Vorteilhaft kann mittels der Ausgangsdaten die auffällige Struktur abgespeichert werden, beispielsweise in einem DICOM-Datensatz, insbesondere als secondary capture.

Die Erfindung betrifft ferner ein computer-implementiertes Verfahren zur Bereitstellung einer trainierten Funktion für ein Röntgensystem aufweisend:

- Empfangen von Eingangstrainingsdaten, wobei sich die Eingangstrainingsdaten auf einen Röntgenaufnahmedatensatz einer Röntgenaufnahme aufweisend mehrere Datenkanäle beziehen,
- Empfangen von Ausgangstrainingsdaten, wobei die Ausgangstrainingsdaten mit den Eingangstrainingsdaten in Verbindung stehen, und wobei die Ausgangstrainingsdaten eine Annotation der Röntgenaufnahme bzw. des Röntgenaufnahmedatensatzes mit einer auffälligen Struktur des Untersuchungsbereichs umfassen,
- Trainieren einer trainierten Funktion basierend auf den Eingangstrainingsdaten und den Ausgangstrainingsdaten,

- Bereitstellen der trainierten Funktion.

Im Schritt des Trainierens wird eine trainierte Funktion basierend auf den Eingangstrainingsdaten und den Ausgangstrainingsdaten mit einer Trainingsrecheneinheit trainiert. Das Trainieren kann enthalten, dass zunächst vorläufige Ausgangsdaten durch Anwenden der trainierten Funktion auf die Eingangstrainingsdaten bestimmt werden und die trainierte Funktion basierend auf dem Vergleich zwischen den zunächst vorläufigen Ausgangsdaten und Ausgangstrainingsdaten angepasst wird.

Die trainierte Funktion kann bereits trainiert sein. Die trainierte Funktion kann alternativ noch nicht trainiert sein. Bei der trainierten Funktion kann es sich um eine noch nicht trainierte Funktion handeln. Das erfindungsgemäße Verfahren kann ein Weitertrainieren der trainierten Funktion umfassen. Beispielsweise kann das Trainieren als ein Anpassungsschritt bzw. -training auf die Eingangstrainingsdaten und die Ausgangstrainingsdaten des Röntgendetektors ausgebildet sein. Die trainierte Funktion kann mit Trainingsdaten mindestens eines anderen Röntgendetektors (vor-)trainiert werden und anschließend im Schritt des Trainierens an den Röntgendetektor angepasst werden.

Die Erfindung betrifft ferner ein erfindungsgemäßes computer-implementiertes Verfahren, wobei die trainierte Funktion durch das erfindungsgemäße computer-implementierte zur Bereitstellung einer trainierten Funktion für ein Röntgensystem bereitgestellt wird, um eine auffällige Struktur des Untersuchungsbereichs in einer Röntgenaufnahme zu ermitteln.

Die Erfindung kann ferner ein Trainingssystem betreffen, aufweisend:

- eine erste Trainingsschnittstelle zum Empfangen von Eingangstrainingsdaten, wobei die Eingangstrainingsdaten auf einen Röntgenaufnahmedatensatz einer Röntgenaufnahme aufweisend mehrere Datenkanäle beziehen,
- eine zweite Trainingsschnittstelle zum Empfangen von Ausgangstrainingsdaten, die Ausgangstrainingsdaten mit den Eingangstrainingsdaten in Verbindung stehen, und wobei die Ausgangstrainingsdaten eine Annotation der Röntgenaufnahme mit einer auffälligen Struktur des Untersuchungsbereichs umfassen,
- eine Trainingsrecheneinheit zum Trainieren einer Funktion basierend auf den Eingangstrainingsdaten und den Ausgangstrainingsdaten,
- eine dritte Trainingsschnittstelle zum Bereitstellen der trainierten Funktion.

In einer besonders vorteilhaften Ausgestaltung kann das Trainingssystem vom Röntgensystem oder der Ermittlungsvorrichtung umfasst sein. Alternativ kann das Trainingssystem mittels eines Netzwerks mit dem Röntgensystem oder der Ermittlungsvorrichtung verbunden sein. Vorteilhaft kann die trainierte Funktion an den Röntgendetektor angepasst werden. Besonders bevorzugt kann durch Anpassung mit neuen Eingangs- und Ausgangstrainingsdaten die trainierte Funktion weiter an den Röntgendetektor angepasst werden.

Die Erfindung betrifft ferner eine Ermittlungsvorrichtung zum Durchführen eines erfindungsgemäßen Verfahrens aufweisend:

- eine erste Schnittstelle zum Empfangen von Eingangsdaten, wobei sich die Eingangsdaten auf einen Röntgenaufnahmedatensatz der Röntgenaufnahme aufweisend mehrere Datenkanäle beziehen,
- eine Rechnereinheit zum Anwenden einer trainierten Funktion auf mindestens zwei Datenkanäle hinsichtlich eines Ermittelns der auffälligen Struktur, wobei Ausgangsdaten erzeugt werden, und wobei die trainierte Funktion auf einem maschinellen Lernverfahren basiert,
- eine zweite Schnittstelle zum Bereitstellen von Ausgangsdaten, wobei die Ausgangsdaten eine auffällige Struktur des Untersuchungsbereichs umfassen.

Die Ermittlungsvorrichtung kann von dem Röntgensystem umfasst sein. Alternativ kann die Ermittlungsvorrichtung ein von dem Röntgensystem entferntes System sein, beispielsweise kann das erfindungsgemäße Verfahren mittels eins cloud-basiertes Datennetzwerks durchgeführt werden. Dazu kann der Röntgenaufnahmedatensatz mittels einer Datenverbindung an die Ermittlungsvorrichtung, insbesondere außerhalb des Kliniknetzwerks, übermittelt werden, dort kann das erfindungsgemäße Verfahren in der Ermittlungsvorrichtung durchgeführt werden und die Ausgangsdaten können zurück in das Kliniknetzwerk oder einen anderen gewünschten Empfänger weitergeleitet werden.

Die Erfindung betrifft ferner ein Röntgensystem aufweisend eine erfindungsgemäße Ermittlungsvorrichtung. Das Röntgensystem kann insbesondere eine Röntgenquelle und einen Röntgendetektor aufweisen. Zwischen der Röntgenquelle und dem Röntgendetektor kann das Untersuchungsobjekt mit dem Untersuchungsbereich angeordnet werden. Bei einer Röntgenaufnahme kann eine Röntgenaufnahmedatensatz erzeugt werden. Dieser kann neben der Röntgenaufnahme bzw. dem Röntgenbild weitere Daten umfassen, beispielsweise Aufnahmeparameter. Das Röntgensystem kann ferner einer Anzeigeeinheit, beispielsweise einen Bildschirm, zum Anzeigen der Ausgangsdaten umfassen. Das Röntgensystem kann ferner eine Eingabeeinheit, beispielsweise eine Tastatur, Maus oder eine andere berührungsempfindliche Eingabeeinheit, zum Eingeben von Benutzereingaben umfassen.

Die Erfindung betrifft ferner ein Computerprogrammprodukt mit einem Computerprogramm, welches direkt in eine Speichereinrichtung einer Steuereinrichtung einer erfindungsgemäßen Ermittlungsvorrichtung oder eines erfindungsmäßen Röntgensystems ladbar ist, mit Programmabschnitten, um alle Schritte eines erfindungsgemäßen Verfahrens auszuführen, wenn das Computerprogramm in der Steuereinrichtung des Röntgensystems ausgeführt wird.

Die Erfindung betrifft ferner ein computerlesbares Medium, auf welchem von einer Rechnereinheit einlesbare und ausführbare Programmabschnitte gespeichert sind, um alle Schritte eines erfindungsgemäßen Verfahrens auszuführen, wenn die Programmabschnitte von der erfindungsgemäßen Ermittlungseinheit oder dem erfindungsgemäßen Röntgensystem ausgeführt werden.

Nachfolgend werden Ausführungsbeispiele der Erfindung anhand von Zeichnungen näher erläutert. Hierbei zeigt:

FIG 1 schematisch eine Darstellung eines erfindungsgemäßen Verfahrens in einer ersten Ausführungsform;

FIG 2 schematisch eine Darstellung eines erfindungsgemäßen Verfahrens in einer zweiten Ausführungsform;

FIG 3 schematisch eine Darstellung eines erfindungsgemäßen Verfahrens in einer dritten Ausführungsform;

FIG 4 schematisch eine Darstellung eines erfindungsgemäßen Verfahrens in einer vierten Ausführungsform;

FIG 5 schematisch eine Darstellung eines erfindungsgemäßen künstlichen neuronales Netzes; und

FIG 6 schematisch eine Darstellung eines erfindungsgemäßen Röntgensystems.

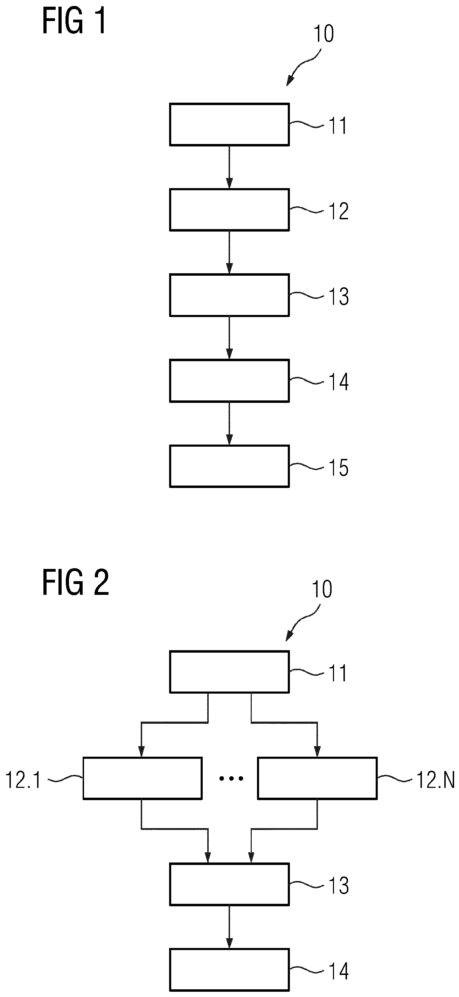

Die Fig. 1 zeigt eine beispielhafte Ausführung eines erfindungsgemäßen Verfahrens 10 in einer ersten Ausführungsform. Das computer-implementiertes Verfahren 10 zum Ermitteln einer auffälligen Struktur in einem Untersuchungsbereich in Verbindung mit einer Röntgenaufnahme eines Röntgensystems weist die Schritte des Empfangens 12, des Anwendens 13 und des Bereitstellens 14 auf. In einem optionalen Schritt des Aufnehmens 11 kann der Röntgenaufnahmedatensatz aufgenommen werden. Der Röntgenaufnahmedatensatz ist mit einem Mehrschichtröntgendetektor oder einem photonenzählender Röntgendetektor aufgenommen. Im Schritt des Empfangens 12 werden die Eingangsdaten empfangen, wobei sich die Eingangsdaten auf einen Röntgenaufnahmedatensatz der Röntgenaufnahme aufweisend mehrere Datenkanäle beziehen. Im Schritt des Anwendens 13 wird eine trainierte Funktion auf die Eingangsdaten angewendet, wobei die trainierte Funktion auf einem maschinellen Lernverfahren basiert, wobei die trainierte Funktion auf mindestens zwei Datenkanäle hinsichtlich eines Ermittelns der auffälligen Struktur angewendet wird, und wobei Ausgangsdaten erzeugt werden. Im Schritt des Bereitstellens 14 werden die Ausgangsdaten bereitgestellt, wobei die Ausgangsdaten eine auffällige Struktur des Untersuchungsbereichs umfassen.

Für jeweils einen Datenkanal kann ein, insbesondere zweidimensionaler, Aufnahmebilddatensatz bereitgestellt werden. Die verschiedenen Datenkanäle können sich auf voneinander verschiedene Datensätze aus der Gruppe beziehen: einem Weichteildatensatz, einem Knochendatensatz, einem nativen Bilddatensatz, einem Kontrastmitteldatensatz, einem Energiebereichsdatensatz oder mehreren voneinander verschiedenen Energiebereichsdatensätzen. Die Eingangsdaten umfassen komplementäre Informationen in den Datenkanälen, spektrale oder materialauflösende Informationen, oder/und Informationen basierend auf einer Rekombination von Bilddaten der Datenkanäle. In einer bevorzugten Ausgestaltung wird die trainierte Funktion gleichzeitig auf die mindestens zwei Datenkanäle angewendet. Die Ausgangsdaten umfassen als auffällige Struktur eine Position einer Läsion, eine Position eines Mikrokalks, eine Position einer Landmarke, einen Abstand oder einen Winkel.

Die Fig. 2 zeigt eine beispielhafte Ausführung eines erfindungsgemäßen Verfahrens 10 in einer zweiten Ausführungsform. Mittels eines Mehrschichtröntgendetektors oder eines photonenzählenden Röntgendetektors kann im Schritt 11 der Röntgenaufnahmedatensatz aufgenommen werden. Der Röntgenaufnahmedatensatz weist mehrere Datenkanäle bzw. mehrere zweidimensionale Aufnahmebilddatensätze auf. Im Schritt des Empfangens 12.1 werden die Informationen eines ersten Datenkanals bzw. des ersten Aufnahmebilddatensatzes als Eingangsdaten empfangen. Im Schritt des Empfangens 12.N werden die Informationen eines N-ten Datenkanals bzw. des N-ten Aufnahmebilddatensatzes als Eingangsdaten empfangen. Auf die Eingangsdaten wird im Schritt des Anwendens 13 die trainierte Funktion, bzw. zumindest eine trainierte Funktion, angewendet und Ausgangsdaten erzeugt. In einem Schritt 14 werden die Ausgangsdaten bereitgestellt.

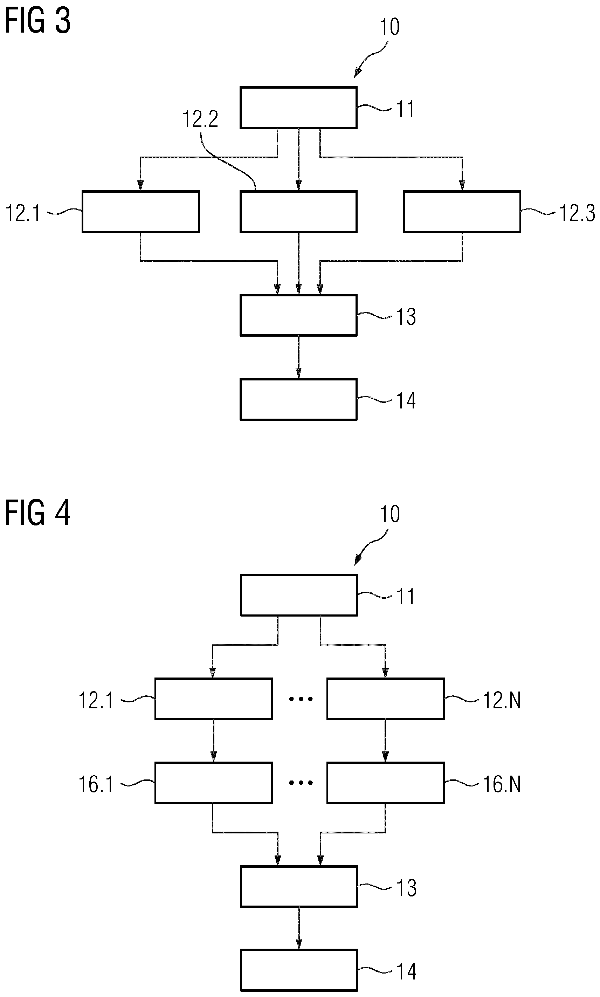

Die Fig. 3 zeigt eine beispielhafte Ausführung eines erfindungsgemäßen Verfahrens 10 in einer dritten Ausführungsform. Die trainierte Funktion basiert auf einem RGB-Modell. Die trainierte Funktion basiert auf einem Deep-Learning-Verfahren für RGB-Bilder. Es werden drei Datenkanäle verwendet. Im Schritt des Empfangens 12.1 wird ein erster Datenkanal bzw. ein erster Aufnahmebilddatensatz, insbesondere ein Weichteildatensatz, empfangen und es wird der erste Datenkanal dem R-Kanal zugeordnet. Im Schritt des Empfangens 12.2 wird ein zweiter Datenkanal bzw. ein zweiter Aufnahmebilddatensatz, insbesondere ein Knochendatensatz, empfangen und es wird der zweite Datenkanal dem G-Kanal zugeordnet. Im Schritt des Empfangens 12.3 wird ein dritter Datenkanal bzw. ein dritter Aufnahmebilddatensatz, insbesondere ein nativer Bilddatensatz, empfangen und es wird der dritte Datenkanal dem B-Kanal zugeordnet. Es können die drei Aufnahmebilddatensätze, welche basierend auf einem Röntgenaufnahmedatensatz eines Mehrschichtröntgendetektors oder eines photonenzählenden Röntgendetektors erzeugt wurden, als Eingangskanäle bzw. Eingangsdaten eines RGB-Modells für einen KI-basierten CAD-Algorithmus bzw. eine solche trainierte Funktion verwendet werden.

Die Fig. 4 zeigt eine beispielhafte Ausführung eines erfindungsgemäßen Verfahrens 10 in einer vierten Ausführungsform. Vor Anwenden 13 der trainierten Funktion wird eine Bildverarbeitung 16.1, ..., 16.N auf den Röntgenaufnahmedatensatz angewendet. Dabei wird die Bildverarbeitung insbesondere separat auf jeden Datenkanal angewendet. Die extrahierten Aufnahmebilddatensätze bzw. Informationen der Datenkanäle können mittels Bildverarbeitung modifiziert werden, bevor sie als Eingangsdaten für die trainierte Funktion verwendet werden.

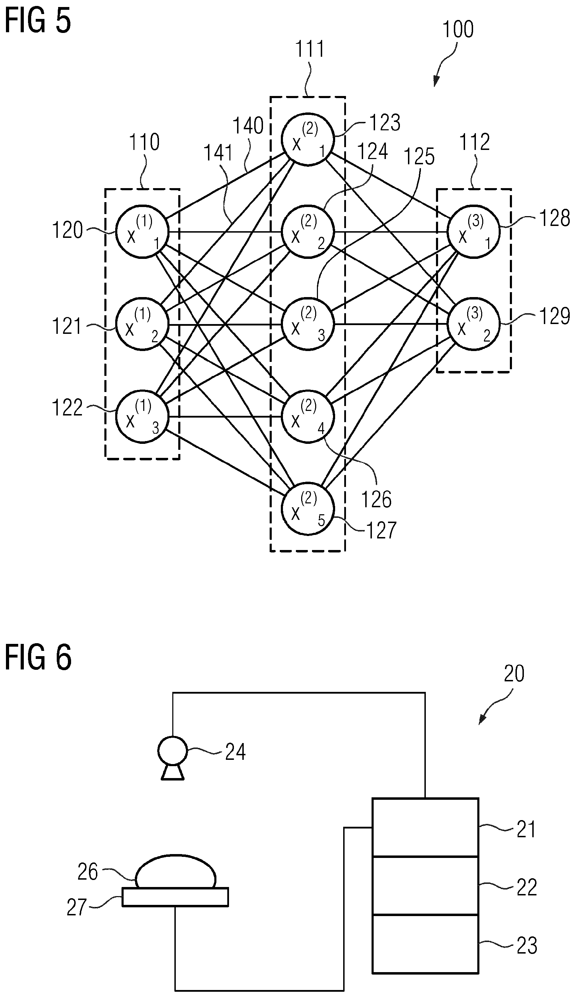

Die Fig. 5 zeigt ein künstliches neuronales Netz 100, wie es im Verfahren gemäß der Figuren 1 bis 4 zum Einsatz kommen kann. Das neuronale Netz kann auch als künstliches neuronales Netz, künstliches neuronales Netzwerk oder neuronales Netzwerk bezeichnet werden.

Das neuronale Netz 100 umfasst Knoten 120,..., 129 und Kanten 140,141, wobei jede Kante 140,141 eine gerichtete Verbindung von einem ersten Knoten 120,..,129 zu einem zweiten Knoten 120,...,129 ist. Im Allgemeinen sind der erste Knoten 120,...,129 und der zweite Knoten 120,...,129 unterschiedliche Knoten, es ist auch möglich, dass der erste Knoten 120,...,129 und der zweite Knoten 120,...,129 identisch sind. Eine Kante 140,141 von einem ersten Knoten 120,...,129 zu einem zweiten Knoten 120,...,129 kann auch als eingehende Kante für den zweiten Knoten und als ausgehende Kante für den ersten Knoten 120,...,129 bezeichnet werden.

Das neuronale Netz 100 antwortet auf Eingabewerte $x^{(1)}_1$, $x^{(1)}_2$, $x^{(1)}_3$ zu einer Vielzahl von Eingangsknoten 120, 121, 122 der Eingangsschicht 110. Die Eingabewerte $x^{(1)}_1$, $x^{(1)}_2$, $x^{(1)}_3$ werden angewendet, um eine oder eine Vielzahl von Ausgaben $x^{(3)}_1$, $x^{(3)}_2$ zu erzeugen. Der Knoten 120 ist beispielsweise über eine Kante 140 mit dem Knoten 123 verbunden. Der Knoten 121 ist beispielsweise über die Kante 141 mit dem Knoten 123 verbunden.

Das neuronale Netz 100 lernt in diesem Ausführungsbeispiel, indem es die Gewichtungsfaktoren $w_{i,j}$ (weights) der einzelnen Knoten basierend auf Trainingsdaten anpasst. Mögliche Eingabewerte $x^{(1)}_1$, $x^{(1)}_2$, $x^{(1)}_3$ der Eingangsknoten 120,121,122 können beispielsweise Schwächungswerte oberhalb einer Schwelle sein, die zuvor aus einem Röntgenaufnahmedatensatz der Röntgenaufnahme, insbesondere einem Datenkanal, extrahiert wurden. Alternativ können die Eingabewerte der Röntgenaufnahmedatensatz der Röntgenaufnahme bzw. die Datenkanäle selbst sein, insbesondere wenn das neuronale Netz 100 ausgebildet ist, auch die Merkmalsextraktion durchzuführen. Beliebige andere Eingabewerte können zur Anwendung kommen.

Das neuronale Netz 100 gewichtet die Eingabewerte der Eingangsschicht 110 basierend auf dem Lernprozess. Die Ausgabewerte der Ausgangsschicht 112 des neuronalen Netzes 100 entsprechen bevorzugt einer auffälligen Struktur, beispielsweise hinsichtlich der Art und/oder Position, sein. Die Ausgabe kann über einen einzelnen oder eine Vielzahl von Ausgabeknoten $x^{(3)}_1$, $x^{(3)}_2$ in der Ausgabeschicht 112 erfolgen.

Das künstliche neuronale Netz 100 umfasst bevorzugt eine versteckte Schicht 111, die eine Vielzahl von Knoten $x^{(2)}_1$, $x^{(2)}_2$, $x^{(2)}_3$ umfasst. Es können mehrere versteckte Schichten vorgesehen sein, wobei eine versteckte Schicht Ausgabewerte einer anderen versteckten Schicht als Eingabewerte verwendet. Die Knoten einer versteckten Schicht 111 verrichten mathematische Operationen. Ein Ausgabewert eines Knotens $x^{(2)}_1$, $x^{(2)}_2$, $x^{(2)}_3$ entspricht dabei einer nicht-linearen Funktion f seiner Eingabewerte $x^{(1)}_1$, $x^{(1)}_2$, $x^{(1)}_3$ und der Gewichtungsfaktoren $w_{i,j}$.

Nach dem Erhalt von Eingabewerten $x^{(1)}_1$, $x^{(1)}_2$, $x^{(1)}_3$ führt ein Knoten $x^{(2)}_1$, $x^{(2)}_2$, $x^{(2)}_3$ eine Summierung einer mit den Gewichtungsfaktoren $w_{i,j}$ gewichteten Multiplikation jedes Eingabewerts $x^{(1)}_1$, $x^{(1)}_2$, $x^{(1)}_3$ durch, wie durch folgende Funktion bestimmt:

$$ x_j^{(n+1)} = f\left(\sum_i x_i^{(n)} \cdot w_{i,j}^{(n)}\right). $$

Der Wichtungsfaktor $w_{i,j}$ kann insbesondere eine reelle Zahl, insbesondere im Intervall von [-1;1] oder [0;1] liegen. Der Wichtungsfaktor $w_{i,j}^{(m,n)}$ bezeichnet das Gewischt der Kante zwischen dem i-ten Knoten einer m-ten Schicht 110,11,112 und einem j-ten Knoten der n-ten Schicht 110,111,112. Der Wichtungsfaktor $w_{i,j}^{(m,n)}$ ist eine Abkürzung für den Wichtungsfaktor $w_{i,j}^{(n,n+1)}$.

Insbesondere wird ein Ausgabewert eines Knotens $x^{(2)}_1$, $x^{(2)}_2$, $x^{(2)}_3$ als Funktion f einer Knoten-Aktivierung, beispielsweise eine Sigmoidalfunktion oder eine lineare Rampenfunktion gebildet. Die Ausgabewerte $x^{(2)}_1$, $x^{(2)}_2$, $x^{(2)}_3$ werden an den bzw. die Ausgabeknoten 128,129 übertragen. Erneut wird eine Summierung einer gewichteten Multiplikation jedes Ausgabewertes $x^{(2)}_1$, $x^{(2)}_2$, $x^{(2)}_3$ als Funktion der Knoten-Aktivierung f und damit die Ausgabewerte $x^{(3)}_1$, $x^{(3)}_2$ berechnet.

Das hier gezeigte neuronale Netz 100 ist ein Feedforward neuronales Netz, bei dem alle Knoten 111 die Ausgabewerte einer vorherigen Schicht in Form ihrer gewichteten Summe als Eingabewerte verarbeiten. Selbstredend können erfindungsgemäß auch andere neuronale Netztypen zum Einsatz kommen, bspw. Feedback-Netze, bei denen ein Eingabewert eines Knotens gleichzeitig auch sein Ausgabewert sein kann.

Das neuronale Netz 100 wird mittels einer Methode des überwachten Lernens trainiert, um Muster zu erkennen. Eine bekannte Vorgehensweise ist die Back-Propagation, die für alle Ausführungsbeispiele der Erfindung angewandt werden kann. Während des Trainings wird das neuronale Netz 100 auf Eingangstrainingsdaten bzw. -werten angewandt und muss entsprechende, vorher bekannte Ausgangstrainingsdaten bzw. -werte erzeugen. Iterativ werden mittlere quadratische Fehler (mean square error - "MSE") zwischen berechneten und erwarteten Ausgabewerten berechnet und einzelne Gewichtungsfaktoren so lange angepasst, bis die Abweichung zwischen berechneten und erwarteten Ausgabewerten unterhalb einer vorbestimmten Schwelle liegt.

Die Fig. 6 zeigt eine beispielhafte Ausführung eines erfindungsgemäßen Röntgensystems 20. Das Röntgensystem 20 umfasst eine Röntgenquelle 24 und einen Röntgendetektor 27, zwischen welchen beiden das Untersuchungsobjekt 26 mit dem Untersuchungsbereich angeordnet ist. Der Röntgendetektor 27 kann insbesondere als Mehrschichtröntgendetektor oder als photonenzählender Röntgendetektor ausgebildet sein. Das Röntgensystem 20 weist eine erfindungsgemäße Ermittlungsvorrichtung auf. Die Ermittlungsvorrichtung zum Durchführen eines erfindungsgemäßen Verfahrens weist eine erste Schnittstelle 21 zum Empfangen von Eingangsdaten, wobei sich die Eingangsdaten auf einen Röntgenaufnahmedatensatz der Röntgenaufnahme aufweisend mehrere Datenkanäle beziehen, auf. Die Die Ermittlungsvorrichtung weist ferner eine Rechnereinheit 22 zum Anwenden einer trainierten Funktion auf mindestens zwei Datenkanäle hinsichtlich eines Ermittelns der auffälligen Struktur auf, wobei Ausgangsdaten erzeugt werden, und wobei die trainierte Funktion auf einem maschinellen Lernverfahren basiert. Die Die Ermittlungsvorrichtung weist ferner eine zweite Schnittstelle 23 zum Bereitstellen von Ausgangsdaten auf, wobei die Ausgangsdaten eine auffällige Struktur des Untersuchungsbereichs umfassen. Die erste und die zweite Schnittstelle können auch eine gemeinsame Schnittstelle sein.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann innerhalb des von den Ansprüchen definierten Schutzumfangs hieraus abgeleitet werden.

**Patentansprüche**

1.  Computer-implementiertes Verfahren (10) zum Ermitteln einer auffälligen Struktur in einem Untersuchungsbereich in Verbindung mit einer Röntgenaufnahme eines Röntgensystems (20) aufweisend folgende Schritte:

    - Empfangen (12) von Eingangsdaten, wobei sich die Eingangsdaten auf einen Röntgenaufnahmedatensatz der Röntgenaufnahme aufweisend mehrere Datenkanäle beziehen,
    - Anwenden (13) einer trainierten Funktion auf die Eingangsdaten, wobei die trainierte Funktion auf einem maschinellen Lernverfahren basiert, wobei die trainierte Funktion auf mindestens zwei Datenkanäle hinsichtlich eines Ermittelns der auffälligen Struktur angewendet wird, und wobei Ausgangsdaten erzeugt werden,
    - Bereitstellen (14) der Ausgangsdaten, wobei die Ausgangsdaten eine auffällige Struktur des Untersuchungsbereichs umfassen,

    **dadurch gekennzeichnet, dass** die Eingangsdaten:

    - komplementäre Informationen in den Datenkanälen,
    - spektrale oder materialauflösende Informationen, oder/und
    - Informationen basierend auf einer Rekombination von Bilddaten der Datenkanäle umfassen.

2.  Verfahren nach einem der vorangehenden Ansprüche, wobei für jeweils einen Datenkanal ein, insbesondere zweidimensionaler, Aufnahmebilddatensatz bereitgestellt wird.

3.  Verfahren nach einem der vorangehenden Ansprüche, wobei sich die Datenkanäle auf voneinander verschiedene Datensätze aus der Gruppe aus:

    - einem Weichteildatensatz, einem Knochendatensatz, einem nativen Bilddatensatz, einem Kontrastmitteldatensatz, einem Energiebereichsdatensatz oder mehreren voneinander verschiedenen Energiebereichsdatensätzen beziehen.

4.  Verfahren nach einem der vorangehenden Ansprüche, wobei der Röntgenaufnahmedatensatz mit einem Mehrschichtröntgendetektor oder einem photonenzählender Röntgendetektor aufgenommen ist.

5.  Verfahren nach einem der vorangehenden Ansprüche, wobei die trainierte Funktion gleichzeitig auf die mindestens zwei Datenkanäle angewendet wird.

6.  Verfahren nach einem der vorangehenden Ansprüche, wobei die trainierte Funktion auf einem Deep-Learning-Verfahren für RGB-Bilder basiert.

7.  Verfahren nach einem der vorangehenden Ansprüche, wobei drei Datenkanäle verwendet werden.

8. Verfahren nach einem der vorangehenden Ansprüche, wobei vor Anwenden der trainierten Funktion eine Bildverarbeitung auf den Röntgenaufnahmedatensatz angewendet wird.

9. Verfahren nach einem der vorangehenden Ansprüche, wobei die Ausgangsdaten als auffällige Struktur eine Position einer Läsion, eine Position eines Mikrokalks, eine Position einer Landmarke, einen Abstand oder einen Winkel umfassen.

10. Ermittlungsvorrichtung zum Durchführen eines Verfahrens nach einem der vorangehenden Ansprüche aufweisend:

    - eine erste Schnittstelle (21) zum Empfangen von Eingangsdaten, wobei sich die Eingangsdaten auf einen Röntgenaufnahmedatensatz der Röntgenaufnahme aufweisend mehrere Datenkanäle beziehen,
    - eine Rechnereinheit (22) zum Anwenden einer trainierten Funktion auf mindestens zwei Datenkanäle hinsichtlich eines Ermittelns der auffälligen Struktur, wobei Ausgangsdaten erzeugt werden, und wobei die trainierte Funktion auf einem maschinellen Lernverfahren basiert,
    - eine zweite Schnittstelle (23) zum Bereitstellen von Ausgangsdaten, wobei die Ausgangsdaten eine auffällige Struktur des Untersuchungsbereichs umfassen,

    **dadurch gekennzeichnet, dass** die Eingangsdaten:

    - komplementäre Informationen in den Datenkanälen,
    - spektrale oder materialauflösende Informationen, oder/und
    - Informationen basierend auf einer Rekombination von Bilddaten der Datenkanäle umfassen.

11. Röntgensystem (20) aufweisend eine Ermittlungsvorrichtung nach Anspruch 10.

12. Computerprogrammprodukt mit einem Computerprogramm, welches direkt in eine Speichereinrichtung einer Steuereinrichtung einer Ermittlungsvorrichtung nach Anspruch 10 oder eines Röntgensystems nach Anspruch 11 ladbar ist, mit Programmabschnitten, um alle Schritte eines Verfahrens nach einem der Ansprüche 1 bis 9 auszuführen, wenn das Computerprogramm in der Steuereinrichtung des Röntgensystems ausgeführt wird.

13. Computerlesbares Medium, auf welchem von einer Rechnereinheit einlesbare und ausführbare Programmabschnitte gespeichert sind, um alle Schritte eines Verfahrens nach einem der Ansprüche 1 bis 9 auszuführen, wenn die Programmabschnitte von der Ermittlungseinheit nach Anspruch 10 oder dem Röntgensystem nach Anspruch 11 ausgeführt werden.

## Claims

1. Computer-implemented method (10) for determining an abnormal structure in an examination region in conjunction with an X-ray recording of an X-ray system (20) having the following steps of:

    - receiving (12) input data, wherein the input data relates to an X-ray recording data set of the X-ray recording having multiple data channels,
    - applying (13) a trained function to the input data, wherein the trained function is based on a machine learning method, wherein the trained function is applied to at least two data channels with regard to determining the abnormal structure, and wherein output data is generated,
    - providing (14) the output data, wherein the output data comprises an abnormal structure of the examination region,
    **characterised in that** the input data comprises:

    - complementary information in the data channels,
    - spectral or material-resolving information or/and
    - information based on a recombination of image data of the data channels.

2. Method according to one of the preceding claims, wherein an, in particular two-dimensional, recording image data set is provided for each data channel.

3. Method according to one of the preceding claims, wherein the data channels relate to data sets that are different from one another from the group of:

    - a soft part data set, a bone data set, a native image data set, a contrast medium data set, an energy region data set or multiple energy region data sets that are different from one another.

4. Method according to one of the preceding claims, wherein the X-ray recording data set is recorded using a multilayered X-ray detector or a photon-counting X-ray detector.

5. Method according to one of the preceding claims, wherein the trained function is applied to the at least two data channels simultaneously.

6. Method according to one of the preceding claims, wherein the trained function is based on a deep learning method for RGB images.

7. Method according to one of the preceding claims, wherein three data channels are used.

8. Method according to one of the preceding claims, wherein prior to applying the trained function an image processing is applied to the X-ray recording data set.

9. Method according to one of the preceding claims, wherein the output data comprises as an abnormal structure a position of a lesion, a position of a microcalcification, a position of a landmark, a distance or an angle.

10. Determining apparatus for implementing a method according to one of the preceding claims having:

    - a first interface (21) for receiving input data, wherein the input data relates to an X-ray recording data set of the X-ray recording having multiple data channels,
    - a computer unit (22) for applying a trained function to at least two data channels with regard to determining the abnormal structure, wherein output data is generated and wherein the trained function is based on a machine learning method,
    - a second interface (23) for providing output data,

    wherein the output data comprises an abnormal structure of the examination region, **characterised in that** the input data comprises:

    - complementary information in the data channels,
    - spectral or material-resolving information or/and
    - information based on a recombination of image data of the data channels.

11. X-ray system (20) having a determining apparatus according to claim 10.

12. Computer program product having a computer program that can be loaded directly into a storage facility of a control facility of a determining apparatus according to claim 10 or an X-ray system according to claim 11 and the computer program product has program sections in order to perform all the steps of a method according to one of claims 1 to 9 if the computer program is executed in the control facility of the X-ray system.

13. Computer readable medium on which program sections, which can be read and executed by a computer unit, are stored in order to perform all the steps of a method according to one of claims 1 to 9 if the program sections are executed by the determining unit according to claim 10 or the X-ray system according to claim 11.

## Revendications

1. Procédé (10) mis en œuvre par ordinateur de détermination d'une structure, qui attire l'attention, dans une zone en examen en liaison avec un enregistrement de rayons X d'un système (20) de rayons X comportant les stades suivants :

    - réception (12) de données d'entrée, dans lequel les données d'entrée se rapportent à un ensemble de données de l'enregistrement de rayons X comportant plusieurs canaux de données,
    - application (13) d'une fonction entraînée aux données d'entrée, dans lequel la fonction entraînée repose sur un procédé d'apprentissage automatique, dans lequel la fonction entraînée est appliquée à au moins deux canaux de données, en ce qui concerne une détermination de la structure, qui attire l'attention, et dans lequel des données de sortie sont produites,
    - mise (14) à disposition des données de sortie, dans lequel les données de sortie comprennent une structure, qui attire l'attention, du domaine à examiner,

    **caractérisé en ce que** les données d'entrée comprennent :

    - des informations complémentaires dans les canaux de données,
    - des informations spectrales ou de dissolution de matière, ou/et
    - des informations reposant sur une recombinaison de données d'image des canaux de données.

2. Procédé suivant l'une des revendications précédentes, dans lequel, pour respectivement un canal de données, on se procure un ensemble de données d'image d'enregistrement, en particulier en deux dimensions.

3. Procédé suivant l'une des revendications précédentes, dans lequel les canaux de données se rapportent à des ensembles de données différents les uns des autres dans le groupe de :

- un ensemble de données de parties molles, un ensemble de données d'os, un ensemble de données d'images natives, un ensemble de données d'agent de contraste, un ensemble de données de plage d'énergie ou plusieurs ensembles de données de plage d'énergie différents les uns des autres.

4. Procédé suivant l'une des revendications précédentes, dans lequel l'ensemble de données d'enregistrement de rayons X est enregistré avec un détecteur de rayons X à plusieurs couches ou un détecteur de rayons X comptant les photons.

5. Procédé suivant l'une des revendications précédentes, dans lequel la fonction entraînée est appliquée simultanément aux au moins deux canaux de données.

6. Procédé suivant l'une des revendications précédentes, dans lequel la fonction entraînée repose sur un procédé d'apprentissage profond pour des images RVB.

7. Procédé suivant l'une des revendications précédentes, dans lequel trois canaux de données sont utilisés.

8. Procédé suivant l'une des revendications précédentes, dans lequel, avant l'application de la fonction entraînée, un traitement d'image est appliqué à l'ensemble de données d'enregistrement de rayons X.

9. Procédé suivant l'une des revendications précédentes, dans lequel les données de sortie comprennent, comme structures, qui attirent l'attention, une position d'une liaison, une position d'un dépôt microcalcique, une position d'un repère, une distance ou un angle.

10. Installation de détermination pour effectuer un procédé suivant l'une des revendications précédentes, comportant :

- une première interface (21) de réception de données d'entrée, dans laquelle les données d'entrée se rapportent à plusieurs canaux de données comportant un ensemble de données de l'enregistrement de rayons X,
- une unité (22) informatique pour l'application d'une fonction entraînée à au moins deux canaux de données en ce qui concerne une détermination de la structure, qui attire l'attention, dans laquelle des données de sortie sont produites, et dans lequel la fonction entraînée repose sur un procédé d'apprentissage automatique,
- une deuxième interface (23) pour la mise à disposition de données de sortie, dans laquelle les données de sortie comprennent une structure, qui attire l'attention, de la zone en examen,

**caractérisée en ce que** les données d'entrée comprennent :

- des informations complémentaires dans les canaux de données,
- des informations spectrales ou de dissolution de matière, ou/et
- des informations reposant sur une recombinaison de données d'image des canaux de données.

11. Système (20) de rayons X comportant une installation de détermination suivant la revendication 10.

12. Produit de programme d'ordinateur comprenant un programme d'ordinateur, qui peut être chargé directement dans un dispositif de mémoire d'un dispositif de commande d'une installation de détermination suivant la revendication 10 ou d'un système de rayons X suivant la revendication 11, comportant des parties de programme pour exécuter tous les stades d'un procédé suivant l'une des revendications 1 à 9, lorsque le programme d'ordinateur est exécuté dans le dispositif de commande du système de rayons X.

13. Support, déchiffrable par ordinateur, sur lequel sont mises en mémoire des parties de programme, déchiffrables et exécutables par une unité informatique, afin d'exécuter tous les stades d'un procédé suivant l'une des revendications 1 à 9, lorsque les parties de programme sont exécutées par l'unité de détermination suivant la revendication 10 ou par le système de rayons X suivant la revendication 11.

## FIG 1

## FIG 2

FIG 3

FIG 4

## FIG 5

## FIG 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 7671342 B2
- US 10957079 B2
- US 20190021677 A1

### In der Beschreibung aufgeführte Nicht-Patentliteratur

- **SANTOS, M. K** ; **FERREIRA JÚNIOR, J. R.** ; **WADA, D. T.** ; **TENÓRIO, A.** ; **BARBOSA, M.,** ; **MARQUES, P.** Artificial intelligence, machine learning, computer-aided diagnosis, and radiomics: advances in imaging towards to precision medicine.. *Radiologia brasileira*, 2019, vol. 52 (6), 387-396, https://doi.org/10.1590/0100-3984.2019.0049
- **L. OAKDEN-RAYNER**. The Rebirth of CAD: How Is Modern AI Different from the CAD We Know?. *Radiology: Artificial Intelligence*, 2019, vol. 1 (3), e180089, https://doi.org/10.1148/ryai.2019180089
- **YANG, H** ; **CONG, W.** ; **WANG, G.** Deep learning for dual-energy X-ray computed tomography. *In Proceedings of The 14th International Meeting on Fully Three-Dimensional Image Reconstruction in Radiology and Nuclear Medicine*, 2017, 864-869
- **LEE, S.** ; **CHOI, Y.H.** ; **CHO, Y.J. et al.** Noise reduction approach in pediatric abdominal CT combining deep learning and dual-energy technique.. *Eur Radiol*, 2021, vol. 31, 2218-2226, https://doi.org/10.1007/s00330-020-07349-9
- **TIANLING LYU** ; **WEI ZHAO** ; **YINSU ZHU** ; **ZHAN WU** ; **YIKUN ZHANG,** ; **YANG CHEN** ; **LIMIN LUO** ; **SHUO LI** ; **LEI XING**. Estimating dual-energy CT imaging from single-energy CT data with material decomposition convolutional neural network. *Medical Image Analysis*, 2021, vol. 70, ISSN 1361-8415, 102001, https://doi.org/10.1016/j.media.2021.102001
- **STEFFEN BRUNS et al.** *Deep Learning from Dual-Energy Information for Whole-Heart Segmentation in Dual-Energy and Single-Energy Non-Contrast-Enhanced Cardiac CT*, https://arxiv.org/abs/2008.03985
- **M. CHEN**. *Automated bone age classification with deep neural networks*, 2016, http://cs231n.stanford.edu/reports/2016/pdfs/310_Report.pd f
- **H. LEE**. Fully automated deep learning systems for bone age assessment. *J Digit Imaging*, 2017, https://link.springer.com/article/10.1007/s10278-017-9955-8
- **S. GUENDEL et al.** ''Learning to Recognize Abnormalities in Chest X-Rays with Location-Aware Dense Networks''. *CIARP*, 2018, https://arxiv.org/abs/1803.04565
- **Z. LI et al.** Thoracic Disease Identification and Localization with Limited Supervision''. *CVPR*, 2018, https://arxiv.org/abs/1711.06373